(19) **Europäisches Patentamt**
**European Patent Office**
**Office européen des brevets**

(11) **EP 3 537 155 B1**

(12) **EUROPEAN PATENT SPECIFICATION**

(45) Date of publication and mention
of the grant of the patent:
**08.03.2023 Bulletin 2023/10**

(21) Application number: **17867676.3**

(22) Date of filing: **02.11.2017**

(51) International Patent Classification (IPC):
*G01N 33/68* (2006.01)    *G01N 33/15* (2006.01)
*G01N 33/50* (2006.01)    *G01N 33/72* (2006.01)

(52) Cooperative Patent Classification (CPC):
**G01N 33/723; G01N 33/6896;** G01N 2333/76;
G01N 2333/805; G01N 2500/00; G01N 2800/2821;
G01N 2800/50

(86) International application number:
**PCT/JP2017/039734**

(87) International publication number:
**WO 2018/084242 (11.05.2018 Gazette 2018/19)**

(54) **METHOD FOR DETERMINING ALZHEIMER'S DISEASE RISK**

VERFAHREN ZUR BESTIMMUNG DES RISIKOS VON MORBUS ALZHEIMER

PROCÉDÉ DE DÉTERMINATION DU RISQUE DE MALADIE D'ALZHEIMER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.11.2016 JP 2016215102**

(43) Date of publication of application:
**11.09.2019 Bulletin 2019/37**

(73) Proprietors:
• **Kyushu University,
National University Corporation
Nishi-ku
Fukuoka-shi
Fukuoka 819-0395 (JP)**
• **Hisayama Research Institute For Lifestyle
Diseases
Kasuya-gun, Fukuoka 811-2501 (JP)**

(72) Inventors:
• **KIYOHARA, Yutaka
Fukuoka-shi
Fukuoka 819-0395 (JP)**
• **NINOMIYA, Toshiharu
Fukuoka-shi
Fukuoka 819-0395 (JP)**
• **MUKAI, Naoko
Fukuoka-shi
Fukuoka 819-0395 (JP)**

• **OHARA, Tomoyuki
Fukuoka-shi
Fukuoka 819-0395 (JP)**
• **KOGA, Masafumi
Amagasaki-shi
Hyogo 661-0953 (JP)**

(74) Representative: **Forstmeyer, Dietmar et al
Boeters & Lieck
Oberanger 32
80331 München (DE)**

(56) References cited:
**EP-A1- 1 876 449      WO-A1-2011/034088
WO-A1-2016/154682    US-A1- 2014 046 160**

• **TOMOE KINOSHITA ET AL: "Association of
GA/HbA1c ratio and cognitive impairment in
subjects with type 2 diabetes mellitus",
JOURNAL OF DIABETES AND ITS
COMPLICATIONS, vol. 30, no. 8, 1 January 2016
(2016-01-01), pages 1452-1455, XP055481748, US
ISSN: 1056-8727, DOI:
10.1016/j.jdiacomp.2016.08.008**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 3 537 155 B1

- **TAKATOSHI IMAI ET AL: "Improved Monitoring of the Hyperglycemic State in Type 1 Diabetes Patients by Use of the Glycoalbumin/HbA1c Ratio", THE REVIEW OF DIABETIC STUDIES, vol. 4, no. 1, 1 January 2007 (2007-01-01) , pages 44-44, XP055622542, DE ISSN: 1613-6071, DOI: 10.1900/RDS.2007.4.44**
- **NAOKO MUKAI ET AL: "Alternative Measures of Hyperglycemia and Risk of Alzheimer's Disease in the Community: The Hisayama Study", JOURNAL OF CLINICAL ENDOCRINOLOGY AND METABOLISM, vol. 102, no. 8, 9 June 2017 (2017-06-09), pages 3002-3010, XP055622545, US ISSN: 0021-972X, DOI: 10.1210/jc.2017-00439**
- **KINOSHITA, T. ET AL.: 'Association of GA/HbAlc ratio and cognitive impairment in subjects with type 2 diabetes mellitus' J DIABETES COMPLICATIONS vol. 30, no. 8, December 2016, pages 1452 - 1455, XP055481748**

## Description

### Technical Field

The present invention relates to a method for measuring glycoalbumin and hemoglobin A1c in a biological sample, and determining the existence or non-existence of a development risk of Alzheimer's disease accurately, simply, rapidly and cheaply from the glycoalbumin/hemoglobin A1c ratio. The present invention also relates to the use of a device and kit for determining the existence or non-existence of a development risk of Alzheimer's disease.

### Background Art

The number of patients with dementia in Japan has risen rapidly in recent years as the population ages. There is concern that the increase in elderly patients with dementia will lead to further increases in medical and nursing care expenses, and significant socioeconomic losses. No fundamental therapies have established for treating dementia, and preventative measures against dementia are important for prolonging healthy lifespans while controlling social security expenses.

Dementia is defined by the Japanese Society of Neurology as a "A condition in which cognitive function having reached normalcy has been continuously lowered due to acquired brain injury and has become an obstacle to everyday life and social life". Dementia is a fundamentally different disease from general cognitive decline because it is predicated on the existence of brain injury and impairment of everyday life and social life.

Diagnostic criteria for dementia that are widely used internationally include the 10th Revision of the International Classification of Diseases (ICD-10) by the World Health Organization (WHO), and the American Psychiatric Association's Diagnostic and Statistical Manual of Mental Disorders, 3rd Edition, Revised (DSM-III-R) and 4th Edition, Text Revision (DSM-IV-TR) (Non-Patent Document 1, p1-2), but actual diagnosis is extremely complicated. As one example, the diagnostic criteria for dementia according to the DMS-III-R are summarized below (Non-Patent Document 1, p2).

A. Impaired (short-term, long-term) memory

B. At least one of the following

1) Impaired abstract thinking

2) Impaired judgment

3) Impaired higher cortical function (Aphasia: language impairment; Apraxia: impaired motor behavior even though motor function is unimpaired; Agnosia: inability to recognize or identify objects even though sensory function is unimpaired; Constructional apraxia: inability to clearly perceive details, grasp relationships among the constituent parts of an object and perform correct synthesis)

4) Personality changes

C. Impairment of A and B interferes with work, social activities or human relationships

D. Not diagnosed when consciousness impaired (eliminate delirium)

E. Presence of organic brain disease can be deduced from medical history and examination

From the above diagnostic criteria for dementia, it can be seen that the pathology of dementia encompasses a variety of diseases, not limited to central nervous system diseases. In the ICD-10, dementia is classified under the heading of "Organic, including symptomatic, mental disorders", and includes four categories: Alzheimer's disease, vascular dementia, dementia in other diseases and unspecified dementia. However, in the DSM-IV-TR dementia is found under the heading of "Delirium, dementia, amnestic disorders and other cognitive disorders", and includes six categories, each of which is further sub-categorized. Many forms of dementia have also been added under the classification of "Dementia due to other general physical disorders" (Non-Patent Document 1, p4-7), including frontotemporal dementia and Lewy body dementia, which have been notable in terms of number of patients in recent years. That is, when speaking of dementia diagnosis by disease type is extremely important because dementia is actually a group of many diseases with a variety of causes, and the symptoms and treatment methods differ according to the disease type. Moreover, since dementia is difficult to cure once it has occurred, it is important to understand the risks and exercise prevention at an early stage. Of the different forms of dementia, there is urgent need for countermeasures against Alzheimer's disease and vascular dementia because of the large number of sufferers.

The DSM-IV and the diagnostic criteria of the National Institute of Neurologic, Communicative Disorders and Stroke and the Alzheimer's Disease and Related Disorders Association (NINCDS-ADRDA) research group are recommended for clinical diagnosis of Alzheimer's disease (Non-Patent Document 1, p225). The most important factors in diagnosing Alzheimer's disease are the "medical history" and "symptomology" (current symptoms), and in the examples of this Description, the Alzheimer's disease diagnostic criteria of the NINCDS-ADRDA were used as described below (Non-Patent Document 2, Non-Patent Document 1, p219).

The specific Alzheimer's disease diagnostic criteria of the NINCDS-ADRDA are as follows.

1) Diagnostic criteria for clinical examination (probable Alzheimer's disease)

- Dementia is identified in clinical testing and in testing by the Mini-Mental State Examination (MMSE), Blessed Dementia Scale or a similar test, and confirmed by neuropsychological testing.

- Deficits in two or more cognitive areas

- Progressive decline in memory and other cognitive functions

- No consciousness impairment

- Onset at age 40 to 90, most common after age 65

- No progressive memory and cognitive impairment attributable to any other systemic disease or brain disease.

2) A diagnosis of 1) probable Alzheimer's disease is supported by the following:

- Progressive impairment in specific cognitive functions: language impairment (aphasia), behavioral impairment (apraxia), impaired recognition (agnosia) and impairment of other activities of daily living (ADL), changes in behavioral patterns, family history of similar impairment. In particular, probable Alzheimer's disease is strongly supported when there is neuropathological confirmation.

- Clinical laboratory findings are confirmed (cerebrospinal fluid normal in normal examination, brain waves normal or exhibit non-specific changes such as increased slow wave activity, progressive cerebral atrophy proved by testing over time using diagnostic imaging such as CT).

3) In some cases, other clinical features consistent with a diagnosis of probable Alzheimer's disease may be confirmed after causes of dementia other than Alzheimer's disease have been excluded.
These "other clinical features" are as follows. Progress may stagnate during the course of the disease, which may be accompanied by symptoms such as depression, insomnia, incontinence, delusions, illusions, hallucinations, violent psychomotor agitation, abnormal sexual behavior and weight loss. Especially in advanced cases, abnormal neurological findings such as increased muscle tone, myoclonus and gait disturbance are seen. Convulsions may occur in advanced cases. CT and other imaging results are normal for the age of the patient.

**4)** Cases in which the diagnosis of probable Alzheimer's disease is doubtful, or features are confirmed that are not characteristic of probable Alzheimer's disease
Sudden stroke attacks, local neurological symptoms, hemiplegia, sensory loss, visual field loss and dyssynergia are seen from the early stage of disease. Convulsive seizures and gait disturbance are confirmed at the time of onset or in the very early stages of disease.

Clinical diagnosis of clinical suspicion (possible Alzheimer's disease)

Dementia symptoms are present and are not attributable to any other neurological, psychological or system disease, but onset, presentation and progress are atypical. There is another systemic disease or brain disorder that could be causative, but is not thought to be the cause of the current dementia.

In a single case of gradually progressing severe dementia, there is no other obvious cause (for research purposes).

The criteria for a definite diagnosis of Alzheimer's disease are based on the clinical diagnostic criteria for probable Alzheimer's disease together with neuropathological evidence from a biopsy or autopsy.

When classifying Alzheimer's disease cases for research purposes, subtypes such as the following are distinguished: familial history, onset before age 65, presence of 21 trisomy, and co-occurrence of other related diseases such as Parkinson's.

It can be understood from the above that Alzheimer's disease is not easy to diagnose, and it can also be extremely difficult to specify risk factors and determine the risk of onset. Therefore, there have long been attempts to find convenient biomarkers.

For example, blood testing is necessary for a diagnosis of exclusion when diagnosing degenerative dementia such as Alzheimer's disease, but in general there are no blood tests for identifying dementia. Low amyloid protein concentration and elevated tau protein concentration in cerebrospinal fluid (CSF) are actively used as surrogate markers for Alzheimer's disease, but cerebrospinal fluid collection is generally difficult to apply to many subjects because it places many burdens on patients. No good indicator for Alzheimer's disease diagnosis has yet been discovered that can be measured in blood samples, which can be collected repeatedly and less invasively than these existing CSF markers (Non-Patent Document 1, p233).

As a result of many observational studies, genetic risk factors (apolipoprotein E$\varepsilon$4), vascular risk factors (high blood pressure, diabetes, high blood cholesterol) and smoking have been identified as risk factors for dementia and Alzheimer's disease, while regular exercise, dietary factors, leisure activities, social participation, active spiritual activity, cognitive training, and moderate alcohol consumption have been identified as preventative factors, but there is little scientific basis for these. At present, no methods have been established for preventing (primary prevention) or delaying the progress (secondary prevention) of Alzheimer's disease (Non-Patent Document 1, p168).

Meanwhile, low amyloid protein concentration and elevated tau protein concentration in cerebrospinal fluid (Non-Patent Document 1, p195) and brain imaging have also been found to be useful for predicting the transition from mild cognitive impairment (MCI) to Alzheimer's disease. It is also thought that morphological imaging (MRI) can be used to predict such transitions with great accuracy based on atrophy of the hippocampus and entorhinal cortex at the start of follow-up (Non-Patent Document 1, p197). However, in the first case testing places a large burden on the patient as discussed above, and is hard to perform on many patients, while in the second case large, expensive equipment is required, which also makes this method hard to apply to large numbers of patients.

The mild cognitive impairment mentioned above is a condition which is not considered dementia because general cognitive function is normal even though there is objective memory impairment. That is, the patient is forgetful, and memory impairment relative to age is confirmed by neuropsychological testing, but this is not considered dementia because ordinary cognitive function is normal and daily living behavior is generally normal (Non-Patent Document 1, p188).

Mild cognitive impairment is important to diagnose because it is considered a precursor state to cognitive decline and dementia, and various ideas have been proposed. Ways of diagnosing mild cognitive impairment include approaches focusing on age-associated memory impairment (AAMI), and those focusing on mild neurocognitive decline (MNCD) caused by neurological disease and physical characteristics.

The criteria for MNCD according to the American Psychiatry Association Diagnostic and Statistical Manual of Mental Disorders, 4th Edition, Text Revision (DSM-IV-TR) are summarized below (Non-Patent Document 1, p14).

1) At least two of the following cognitive disorders are present, and have been present almost continuously for at least 2 weeks

    1. Memory impairment identified as reduced ability to learn or remember information

    2. Executive function impairment

    3. Attention or information processing speed impairment

    4. Sensory or motor function decline

    5. Language impairment (comprehension, paraphrasing)

2) Objective evidence of neurological or general physical disease judged to be a cause of cognitive impairment is found from physical examination or clinical laboratory findings (including neuroimaging)

3) There is evidence of behavioral abnormality or deterioration from neuropsychological evaluation or quantitative cognitive evaluation

4) Cognitive impairment causes severe distress or functional impairment in an important social, professional or other area, and has declined from a previous level

5) Cognitive impairment does not meet the criteria for delirium, dementia or amnestic disorder, and cannot be explained by other mental disorder

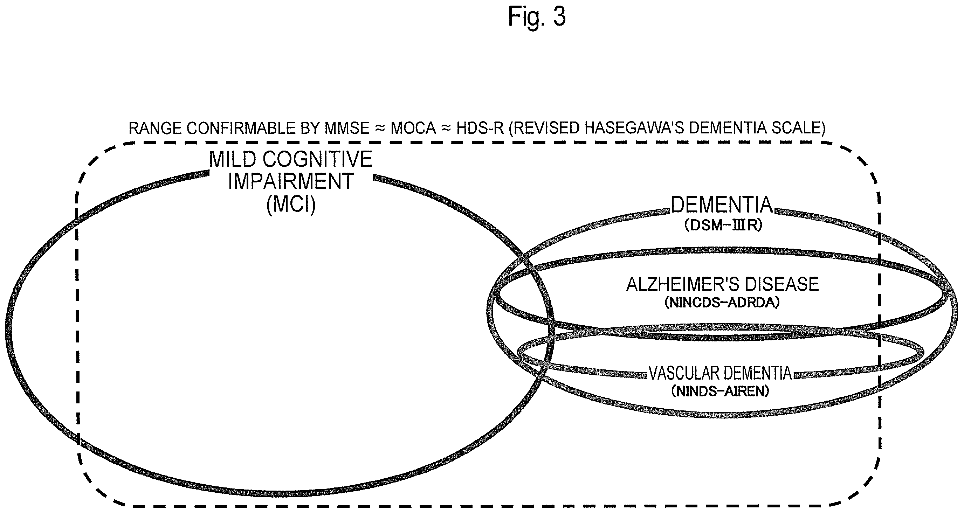

To summarize, mild neurocognitive decline and dementia are two entirely different conditions, while dementia itself encompasses many pathologies, and Alzheimer's disease in particular is a disease type with many patients (Fig. 3). Furthermore, it is extremely difficult to diagnose or determine the risk of developing Alzheimer's disease, and there is strong demand for an accurate, simple, rapid and inexpensive method, biomarker, kit and device for determining Alzheimer's disease onset or onset risk. There is also demand for development of compounds useful for treating and preventing Alzheimer's disease.

Meanwhile, diabetes is increasing rapidly worldwide. It is well known that diabetes causes arteriosclerosis and can cause or promote cerebrovascular disorders, and an association with vascular dementia is expected. Moreover, epidemiological studies in Europe and the US have pointed to an association between diabetes and Alzheimer's disease.

the Hisayama-machi study is an epidemiological survey of typical lifestyle diseases in Japan, and includes a long-term epidemiological survey of dementia in elderly patients aged 65 and older. The Hisayama-machi study is a cohort study (prospective follow-up study) of local residents in Hisayama-machi in Fukuoka Prefecture, and the prevalence of dementia was studied between 1985 and 2012. The examination rate is over 90% and 1904 subjects were examined in 2012, so this is a highly reliable study involving a large number of subjects. In the follow-up to this study, Alzheimer's disease was significantly more prevalent in the diabetic group, while vascular dementia was significantly more prevalent in the pre-diabetic impaired glucose tolerance (IGT) group and the diabetic group, and a significant association between diabetes and Alzheimer's disease onset (hazard ratio 2.1) persisted even when other risk factors for dementia were adjusted by multivariate analysis (Non-Patent Document 3). When the subjects were classified based on postprandial blood glucose and blood glucose 2 hours after glucose challenge, the risk of Alzheimer's disease and vascular dementia were shown to rise linearly as the blood glucose levels rose (Non-Patent Document 3). Thus, the association between diabetes and dementia has been studied, but there has been no study of an association with glycoalbumin and hemoglobin A1c.

Glycated proteins such as hemoglobin A1c and glycoalbumin are commonly measured clinically for purposes of diabetes diagnosis and glycemic control, and are widely used as indicators of glycemic control. Hemoglobin A1c is a substance comprising glucose bound to hemoglobin in red blood cells, and considering that the lifespan of red blood cells in blood is about 120 days, it is considered to be an indicator reflecting glycemic control status over the course of about 2 to 3 months. Glycoalbumin, on the other hand, is a substance comprising glucose bound to albumin in blood, and considering that the half-life of albumin in blood is about 17 days, it is used as an indicator representing glycemic control status over 2 to 3 weeks. That is, glycoalbumin is a marker representing glycemic control status over a shorter period than hemoglobin A1c.

The JDS (Japan Diabetes Society) values set by the Japan Diabetes Society have conventionally been used as hemoglobin A1c values, but international standards were adopted in April of 2012, and the values set by the NGSP (National Glycohemoglobin Standardization Program) in the US are now used. The following formula shows the relationship between the JDS value and NGSP value, and the JDS values used in prior literature can be converted using this formula.

NGSP value (%) = 1.02 x JDS value (%) + 0.25%          Formula 1

JDS value 4.9% or less: NGSP value (%) = JDS value (%) + 0.3%   Formula 2

JDS value 5.0% to 9.9%: NGSP value (%) = JDS value (%) + 0.4% Formula 3

JDS value 10.0% to 14.9%: NGSP value (%) = JDS value (%) + 0.5%          Formula 4

Glycohemoglobin means the same thing as hemoglobin A1c. The Glycoalbumin/hemoglobin A1c ratio is an important indicator in the present invention, and when characteristic values such as cut-off values are described in this Description they refer to NGSP values. However, when citing numbers based on prior literature, the JDS value and NGSP values are distinguished by writing hemoglobin A1c (JDS) or hemoglobin A1c (NGSP).

The NGSP hemoglobin A1c values are not used in Europe, and instead the IFCC (International Federation of Clinical Chemistry and Laboratory Medicine) values are used. The IFCC values can be converted into NGSP values by using the following conversion formula.

$$\text{NGSP value} = (0.09148 \times \text{IFCC value}) + 2.152 \qquad \text{Formula 5}$$

Thus, hemoglobin A1c values are represented in various ways, but since these are convertible they can be read appropriately as NGSP values.

Like hemoglobin A1c, glycoalbumin has also been a target of standardization particularly by the Japan Society of Clinical Chemistry. The current units are HPLC-traceable percentage values currently used in Japan, Europe, the US, China and the like, but research is currently being done into future standardization to isotope dilution mass spectrometry (ID/MS) (unit: mmol/mol) methods using glycolysine and lysine as primary standard substances (Patent Document 4). These units can be converted using the following Formula 6 and the Formulas 7 to 9 developed in-house by Asahi Kasei, but the coefficients may vary slightly depending on the test conditions and the like, and conversion can also be accomplished using formula determined in future conferences and papers, or using formula established by various companies. In this Description, the numbers for glycoalbumin (GA, %) are widely-used HPLC-traceable percentage values.

$$\text{GA (\%)} = 0.0523 \times \text{GA (mmol/mol)} + 1.306 \qquad \text{Formula 6}$$

$$\text{GA (\%)} = 0.0536 \times \text{GA (mmol/mol)} + 0.3040 \qquad \text{Formula 7}$$

$$\text{GA (\%)} = 0.0539 \times \text{GA (mmol/mol)} + 0.5839 \qquad \text{Formula 8}$$

$$\text{GA (\%)} = 0.0545 \times \text{GA (mmol/mol)} + 0.4811 \qquad \text{Formula 9}$$

It is also known that the values for hemoglobin A1c (JDS) and glycoalbumin each assume steady values and the glycoalbumin/hemoglobin A1c (JDS) ratio assumes a characteristic value of about 3 when blood glucose is maintained at a stable, fixed level for a sufficiently long time, such as about 3 months. When the glycemic control status changes, however, the ratio varies from the value of about 3 because the glycoalbumin changes first. Because glycoalbumin becomes elevated first when the glycemic control status deteriorates, the glycoalbumin/hemoglobin A1c (JDS) ratio rises above the characteristic value of about 3, and falls below about 3 when the glycemic control status improves. Consequently, it is possible to judge from the glycoalbumin/hemoglobin A1c (JDS) ratio that blood glucose has recently risen or fallen.

Furthermore, the glycoalbumin/hemoglobin A1c (JDS) ratio is known to rise above the characteristic value of about 3 when daily fluctuations in blood glucose are large, even if the glycemic control status is stable (Non-Patent Document 5). For example, comparing type 1 diabetic patients with severe blood glucose fluctuations and type 2 diabetic patients with relatively small fluctuations, high glycoalbumin/hemoglobin A1c (JDS) ratios have been reported in the former group. It has also been reported that blood glucose fluctuations can be predicted from the divergence of the glycoalbumin/hemoglobin A1c (JDS) ratio, and it is hypothesized that when blood glucose fluctuations are large in type 2 diabetic patients, the glycoalbumin/hemoglobin A1c (JDS) ratio rises above the characteristic value of 3.

Regarding associations with diabetic complications, it has been reported that the glycoalbumin/hemoglobin A1c (JDS) ratio is associated with diabetic nephropathy (Non-Patent Document 5), and can be used to predict the risk of developing Alzheimer's disease or vascular dementia in cases of acute cerebral infarction (Patent Document 1), and in a method for testing kidney function in diabetic patients (Patent Document 2). Moreover, in diseases such as liver cirrhosis, kidney failure, thyroid disease and anemia that affect the lifespan of hemoglobin and the half-life of albumin, the glycoalbumin/hemoglobin A1c (JDS) ratio reportedly diverges from the characteristic value of about 3 irrespective of fluctuations in blood glucose.

Regarding associations with cognitive function, a study has been reported (Non-Patent Document 6) regarding an association between the glycoalbumin/hemoglobin A1c (NGSP) ratio and cognitive disorder in elderly Chinese patients. In this study, when the subjects were separated into two groups having glycoalbumin/hemoglobin A1c (NGSP) ratios of 2.53 or above and under 2.53, the scores of the high-value group were significantly lower on the Mini-Mental State Examination (MMSE) and Montreal Cognitive Assessment (MoCA), with are tests for evaluating cognitive function.

The results of multiple regression analysis showed that the MMSE and MoCA scores were inversely correlated with glycoalbumin and the glycoalbumin/hemoglobin A1c (NGSP) ratio, but did not correlate with hemoglobin A1c (NGSP). This is an extremely interesting study, but the aforementioned mild cognitive impairment (MCI) is considered to be a disease with an entirely different concept from Alzheimer's disease, which is a degenerative disease of the central nervous system (see Fig. 3). That is, this document does not describe Alzheimer's disease and the glycoalbumin/hemoglobin A1c ratio, and does not investigate predicting the onset or risk of developing Alzheimer's disease based on the glycoalbumin/hemoglobin A1c ratio.

In Non-Patent Document 7, when 35 type 2 diabetes patients aged 55 and older were assessed with the revised Hasegawa's Dementia Scale (HDS-R), the average glycoalbumin/hemoglobin A1c ratio was $3.1 \pm 0.9$, and the possibility of using this ratio as an indicator to predict dementia is suggested in the document. However, the value of $3.1 \pm 0.9$ is not a cut-off value showing that "cognitive function has declined" or "Alzheimer's disease is present", but merely represents an average $\pm$ SD value for 35 subjects. The results of statistic analysis in this document show an inverse correlation between the scores on the Hasegawa scale and the glycoalbumin/hemoglobin A1c ratio, but this only means that the glycoalbumin/hemoglobin A1c ratios were higher the lower the score on the Hasegawa scale, and Alzheimer's disease and dementia cannot be diagnosed simply on this basis.

To summarize, the glycoalbumin/hemoglobin A1c ratio has been reported to be not simply an indicator of blood glucose status, but also to diverge from the characteristic value of about 3 in various pathologies when hemoglobin A1c (JDS) is used, and various pathologies can be evaluated based on the degree of divergence, but there have so far no association has been reported between Alzheimer's disease and the glycoalbumin/hemoglobin A1c ratio. Although an association has been reported between cognitive disorder and the glycoalbumin/hemoglobin A1c ratio, however, there have been no studies of Alzheimer's disease focusing on the glycoalbumin/hemoglobin A1c ratio, and no studies in which Alzheimer's disease onset was evaluated or Alzheimer's disease risk was assessed using the glycoalbumin/hemoglobin A1c ratio.

Citation List

Non-Patent Documents

Non-Patent Document 1: 2010 Guideline for Treatment of Dementia Disease (October 15, 2010), Supervisor: Japanese Society of Neurology, Editor: "Guideline for Treatment of Dementia Disease" Joint Working Committee, Issued by: Igaku-Shoin Ltd.

Non-Patent Document 2: Guy McKhann et al., Clinical diagnosis of Alzheimer's disease, Neurology 1984, 34:939-944

Non-Patent Document 3: Tomoyuki Kohara and Yutaka Kiyohara, Dementia in diabetes patients, Gekkan Tonyobyo (Diabetes Monthly), 2012, 4:12-20

Non-Patent Document 4: Izumi Takei et al., Recommended JSCC methods for measuring glycoalbumin, Rinshokagaku (Clinical Chemistry), 2008, 37:178-191

Non-Patent Document 5: Kwang Joon Kim et al., The roles of glycated albumin as intermediate glycation index and pathogenic protein, Diabetes Metab. J. 2012, 36:98-107

Non-Patent Document 6: Yuan Zhong et al., GA to HbA1C ratio, but not HbA1C is associated with cognition in Chinese nondiabetic old adults, Aging Mental Health, 2015, 19(9):853-857

Non-Patent Document 7: Tomoe Kinoshita et al., Association of GA/HbA1c ratio and cognitive impairment in subjects with type 2 diabetes mellitus, J. Diabetes Complications, 2016 doi, 10.1016/j.jdiacomp.

Patent Documents

Patent Document 1: WO 2011/034088
Patent Document 2: Patent Publication JP-A-2015-96835

## Summary

Technical Problem

It is an object of the present invention to provide a method and a use of the glycoalbumin/hemoglobin A1c ratio for determining the existence or non-existence of a development risk of Alzheimer's disease accurately, simply, rapidly and cheaply, as well as use of a kit and a device for determining whether there is a risk of developing Alzheimer's disease.

Solution to Problem

As part of a cohort study (prospective follow-up study) of local residents of Hisayama-machi, the inventors administered a 75 g oral glucose tolerance test to 1017 elderly subjects in 1988, and followed these subjects for 15 years in order to observe the effects of abnormal glucose metabolism on dementia including Alzheimer's disease and vascular dementia (during the follow-up period 232 subjects developed dementia, and 209 of these cases were investigated morphologically by either brain autopsy or CT/MRI imaging). As a result, diabetes, and particularly blood glucose 2 hours after challenge (postprandial hyperglycemia) was found to correlate significantly with development of Alzheimer's disease. In an attempt to discover biomarkers for predicting Alzheimer's disease onset more easily than with the complicated 75 g oral glucose tolerance test, the inventors also investigated the association between levels of serum glycoalbumin, hemoglobin A1c and 1,5-anhydroglucitol measured at the start of follow-up in 1187 elderly patients who were examined in 2007 and the risk of dementia by disease type during the subsequent 5-year follow-up period. Looking at the results, unexpectedly no correlation was found between these indicators and Alzheimer's disease or vascular dementia, but interestingly, only the glycoalbumin/hemoglobin A1c (NGSP) ratio emerged as a statistically significant risk factor in the development of Alzheimer's disease. When the study then looked at different levels of glycoalbumin/hemoglobin A1c ratios, it was found that the risk of developing Alzheimer's disease was statistically significantly higher when the ratio was at least 2.6.

That is, the inventors achieved the present invention after measuring glycoalbumin and hemoglobin A1c in biological samples, and discovering that by determining the glycoalbumin/hemoglobin A1c ratio, it was possible to determine the existence or non-existence of a development risk of Alzheimer's disease. Up to now, there have been no reports of studies associating the glycoalbumin/hemoglobin A1c ratio with determining the existence or non-existence of a development risk of Alzheimer's disease, and the present invention is based on the new and groundbreaking discovery that the existence or non-existence of a development risk of Alzheimer's disease can be determined easily, rapidly and cheaply by using the glycoalbumin/hemoglobin A1c ratio. The following inventions are provided by the present invention.

[1] A method for determining the existence or non-existence of a development risk of Alzheimer's disease, comprising a step of determining that a subject is at risk of developing Alzheimer's disease when a glycoalbumin/hemoglobin A1c ratio is at least 2.6 in a blood sample from the subject.

[2] The method according to [1], wherein the blood sample is subjected to the following steps (1) to (3) prior to the step of determining the incidence of or the existence or non-existence of a development risk of Alzheimer's disease:

1) measuring glycoalbumin and hemoglobin A1c in the blood sample from a subject;
2) using the measurement values obtained in step 1) to calculate the glycoalbumin/hemoglobin A1c ratio; and
3) comparing the glycoalbumin/hemoglobin A1c level obtained in step 2) with a cut-off value for predicting the incidence of or the existence or non-existence of a development risk of Alzheimer's disease.

[3] A method according to [1] or [2], wherein the glycoalbumin/hemoglobin A1c ratio is at least 2.85.
[4] A method according to any of [1] to [3], wherein the subject does not suffer from mild cognitive impairment (MCI).
[5] A method according to any of [1] to [4], wherein the subject does not suffer from vascular dementia.
[6] Use of a biomarker for determining the existence or non-existence of a development risk of Alzheimer's disease, wherein a glycoalbumin/hemoglobin A1c ratio is used as an indicator, and wherein a subject is determined to be at risk of developing Alzheimer's disease when the glycoalbumin/ hemoglobin A1c ratio in a blood sample from the subject is at least 2.6.
[7] Use of a device for determining the existence or non-existence of a development risk of Alzheimer's disease, having

(a) a calculation part for calculating a glycoalbumin/hemoglobin A1c ratio based on a measured glycoalbumin value and hemoglobin A1c value, and
(b) an evaluation part for evaluating the existence or non-existence of a development risk of Alzheimer's disease based on the calculated glycoalbumin/hemoglobin A1c ratio.

[8] The use of a device according to [7], further comprising a measuring part for glycoalbumin and/or a measuring part for hemoglobin A1c.

[9] Use of a kit for determining the existence or non-existence of a development risk of Alzheimer's disease, containing a glycoalbumin measurement reagent and a hemoglobin A1c measurement reagent, and further containing a manual stating to the effect that a subject is determined to be suffering from Alzheimer's disease or at risk of developing Alzheimer's disease when the glycoalbumin/hemoglobin A1c ratio is at least 2.6.

Advantageous Effects of Invention

Alzheimer's disease could not be identified specifically by conventional methods in a simple way. However, the present invention allows the existence or non-existence of a development risk of Alzheimer's disease to be determined accurately, easily, rapidly and cheaply.

**Brief Description of Drawings**

Fig. 1 shows the hazard ratios for Alzheimer's disease onset according to the levels of blood glucose-associated indicators based on Example 1 of the present invention.

Fig. 2 shows hazard ratios of blood glucose-associated indicator levels for Alzheimer's disease onset according to the presence or absence of abnormal glucose metabolism (diabetes + prediabetes) based on Example 2 of the present invention.

Fig. 3 shows the relationship between Alzheimer's disease and other diseases, and the relationships between these diseases and various measurement methods. Mild cognitive impairment (MCI) here means a condition in which multiple cognitive functions having reached normalcy are then lowered by some cause, but without becoming an obstacle to everyday life and social life. The disease concept of MCI and the disease concepts of mild neurocognitive decline (MNCD) according to the DSM-4 and mild neurocognitive disorder (MNCD) according to the DSM-5 are all similar. On the other hand, dementia is a condition in which either mild cognitive impairment or more serious cognitive decline is present and becomes an obstacle everyday life or social life. Types of dementia include Alzheimer's disease and vascular dementia. The diagnostic criteria for dementia used in the present study are the DSM-IIIR criteria for dementia, the NINCDS-ADRDA criteria for Alzheimer's disease and the NINDS-AIREN criteria for vascular dementia. Moreover, the MMSE, MoCA and revised Hasegawa's Dementia Scale (HDS-R) are tests of cognitive function, and are all three equivalent (MMSE $\approx$ MoCA $\approx$ HDS-R). The range of cognitive decline that can be confirmed with these tests is limited to MCI and dementia, and no one test can identify all diseases. However, the results of the present study can specifically identify Alzheimer's disease.

Fig. 4 is a block diagram showing the functional configuration of a device for determining the incidence of or the existence or non-existence of a development risk of Alzheimer's disease in the present invention.

**Description of Embodiments**

The present invention is explained in more detail below and the scope of the invention is defined by the appended claims.

(1) Glycoalbumin

Glycoalbumin in the present invention is a form of albumin comprising glucose bound non-enzymatically to albumin, and may be called glycoalbumin or glycated albumin. Glycoalbumin can be measured by HPLC or by immune methods or enzymatic methods for example. Currently, enzymatic methods are widely used for measuring glycoalbumin. Glycoalbumin measurement in the present invention includes not only measuring the glycoalbumin concentration in a biological sample, but also measuring the glycoalbumin concentration as a percentage of the total albumin in a biological sample.

In enzymatic methods, for example the glycoalbumin in a biological sample is first digested with a protease to produce glycated amino acids. These glycated amino acids are then reacted with ketoamine oxidase, and the resulting hydrogen peroxide is reacted with a peroxidase in the presence of a hydrogen donor and a dye to quantify the glycated amino acids. The glycoalbumin concentration in a biological sample can also be determined from the quantified glycated amino acids by using a known calibrator or the like. The albumin concentration in the same biological sample is determined by an albumin quantification method such as an improved BCP assay, and the glycoalbumin concentration is divided by the albumin concentration to determine the percentage (%) of glycoalbumin to total albumin. In Japan, standard methods for measuring glycoalbumin are recommended by the Japan Society of Clinical Chemistry (Non-Patent Document 4), and it is desirable to use sufficiently accurate and precise measurement methods conforming to standard methods of glycoalbumin measurement.

In this Description, glycoalbumin is represented as an HPLC-traceable value (%), but when using another unit or a value traceable to another method, these can be converted appropriately using Formula 6 above or the like. Moreover, glycoalbumin is sometimes abbreviated as GA in the present invention.

(2) Hemoglobin A1c

In the present invention, hemoglobin A1c is a form of hemoglobin comprising glucose non-enzymatically bound to the N-terminal valine of the hemoglobin β chain, and may be called hemoglobin A1c, HbA1c, glycohemoglobin, glycohemoglobin A1c, A1C or the like. Hemoglobin A1c can be measured by HPLC or by an immune method or enzymatic method for example. At present, HPLC methods, immune methods and enzymatic methods are all widely used for measuring hemoglobin A1c. Hemoglobin A1c measurement in the present invention includes not only measuring the hemoglobin A1c in a biological sample, but also measuring the hemoglobin A1c as a percentage of the total hemoglobin in the biological sample.

In HPLC methods, hemoglobin A1c in which the N-terminal valine of the β chain is glycated can be separated and measured with a cation exchange column for example, and the percentage (%) of hemoglobin A1c relative to total hemoglobin in the biological sample can be determined. In immunological methods, a peptide bound to the glucose of the β chain N-terminal valine is used as an antigen, and measurement is performed with the resulting antibody. In enzymatic methods, a glycated peptide excised using a protease that cleaves the peptide at the N-terminal of the β chain is conducted to a color development system using fructosyl peptide oxidase to quantify the hemoglobin A1c. Hemoglobin A1c measurement values have been standardized by the Japan Diabetes Society, the Japan Society of Clinical Chemistry and the US National Glycohemoglobin Standardization Program (NGSP) and the like, and it is desirable to use sufficiently accurate and precise measurement methods conforming to standard methods. In this Description, glycoalbumin is represented as an NGSP-traceable value (%), but when using another unit or a value traceable to another method, these can be converted appropriately using Formula 1 to 5 or the like above. Moreover, hemoglobin A1c is sometimes abbreviated as HbA1c or A1C in the present invention.

The glycoalbumin/hemoglobin A1c ratio in the present invention is a ratio obtained by dividing the measured glycoalbumin value by the measured hemoglobin A1c value.

(3) Determining the incidence of or risk of developing Alzheimer's disease

Determining the incidence of or the existence or non-existence of a development risk of Alzheimer's disease means the act of evaluating the risk (likelihood) that a subject currently suffers from Alzheimer's disease and the risk (likelihood) of developing Alzheimer's disease when the subject does not suffer from Alzheimer's disease but may develop it in the future. The incidence of or the existence or non-existence of a development risk of Alzheimer's disease is normally determined by statistical methods as an odds ratio or hazard ratio showing increased frequency given 1 as the risk when a certain item is normal.

The Mini-Mental State Examination (MMSE), Montreal Cognitive Assessment (MoCA) and revised Hasegawa's Dementia Scale (HDS-R) can only identify mild cognitive impairment (MCI) and some forms of dementia. On the other hand, the present invention can identify Alzheimer's disease specifically. That is, the Alzheimer's disease targeted by the present invention is distinguished from mild cognitive impairment (MCI) or vascular dementia (Fig. 3). The disease concept of mild cognitive impairment and the disease concepts of mild neurocognitive decline (MNCD) according to the DSM-4 and mild neurocognitive disorder (MNCD) according to the DSM-5 are all almost identical.

The present invention provides a method for predicting the existence or non-existence of a development risk of Alzheimer's disease using the glycoalbumin/hemoglobin A1c ratio in a biological sample as an indicator. The present invention may be implemented alone or in combination with another method. For example, using the method of the present invention together with a commonly-used method of determining the existence or non-existence of a development risk of Alzheimer's disease is desirable from the standpoint of increasing the prediction accuracy.

In commonly-used methods, the incidence of or the existence or non-existence of a development risk of Alzheimer's disease is determined by considering low amyloid protein concentrations and elevated tau protein concentration in cerebrospinal fluid (CSF), genetic risk factors (apolipoprotein Eε4), vascular risk factors (high blood pressure, diabetes, hypercholesterolemia) and smoking and the like, and also after considering such protective factors as regular exercise, dietary factors, leisure activities, social participation, active spiritual activity, cognitive training and moderate alcohol consumption.

A cut-off value for determining the existence or non-existence of a development risk of Alzheimer's disease is explained in detail below.

It is generally known that a glycoalbumin/hemoglobin A1c (JDS) ratio of about 3 in blood from a diabetes patient is a characteristic value when blood sugar is stabilized (Non-Patent Document 5).

In the present invention, glycoalbumin/hemoglobin A1c (NGSP) ratios were determined from measured values for glycoalbumin and hemoglobin A1c (NGSP), and these ratios were compared with separately performed Alzheimer's disease diagnoses, and the relationship was investigated. The characteristic values of about 2 to 4 for glycoalbumin/hemoglobin A1c (NGSP) ratio may vary depending on race, gender, age and target population, and the measurement units and the like may also change in the future due to international standardization.

A cut-off value for determining the existence or non-existence of a development risk of Alzheimer's disease is a reference value for glycoalbumin/hemoglobin A1c ratio used to assess the (percentage) likelihood that a subject will suffer in the future if they do not at present, or will develop Alzheimer's disease in the future. There may be single cut-off value or multiple cut-off values.

Any number of glycoalbumin/hemoglobin A1c (NGSP) ratio of at least 2.6 or above such as between 2.6 and 3.2 or preferably between 2.7 and 3.1 can be used as a cut-off value. The values of 2.85 or 2.83 shown in the examples may be used, but since these values may change slightly under examination, any numbers near these numbers may be used.

The glycoalbumin/hemoglobin A1c (JDS) ratios of healthy subjects and diabetes patients with stable blood glucose are characteristically about 3.0 (or 2.83 or 2.85 using the NGSP values in the examples of this application), and since the glycoalbumin/hemoglobin A1c (NGSP) ratio diverges from the characteristic values as the likelihood of the existence or non-existence of a development risk of it in the future increases, a cut-off value may be 1 or more numbers selected from 2.6, 2.7, 2.8, 2.83, 2.85, 2.9, 3.0, 3.1 and 3.2, or another value may be used.

Alternatively, in at least 20 (or preferably at least 30, or more preferably at least 50, or most preferably at least 70) patients, a cut-off value can be obtained by measuring glycoalbumin and hemoglobin A1c (NGSP), determining the glycoalbumin/hemoglobin A1c (NGSP) ratios, and comparing these with the results for healthy subjects not suffering from Alzheimer's disease and cognitive syndrome according to the NINCDS-ADRDA diagnostic criteria for Alzheimer's disease. A cut-off value can also be obtained by known statistical methods (ROC analysis, etc.) (Examples). Since a cut-off value of 2.85 for determining the existence or non-existence of a development risk of Alzheimer's disease was obtained by such methods in the Examples of this Description, a cut-off value of 2.85 is preferably selected for evaluating the existence or non-existence of a development risk of Alzheimer's disease.

The comparison of measured glycoalbumin/hemoglobin A1c (NGSP) ratios with a cut-off value for determining the existence or non-existence of a development risk of Alzheimer's disease, and the determination of the existence or non-existence of a development risk of Alzheimer's disease based on this comparison, are explained in detail below.

When the cut-off value for determining the existence or non-existence of a development risk of Alzheimer's disease is a single number, it is possible to predict that a subject is at high risk of developing Alzheimer's disease in the future even if they do not have it at present if the ratio of the measured values for glycoalbumin and hemoglobin A1c in a biological sample from that subject is at or above the cut-off value, and to predict a low likelihood of Alzheimer's disease when the ratio is below the cut-off value.

For example, if the cut-off value for determining the existence or non-existence of a development risk of Alzheimer's disease is 2.85, there is a high risk that the subject may develop it in the future even if they do not have it now if the glycoalbumin/hemoglobin A1c (NGSP) ratio measured in a biological sample is 2.85 or more, while if the ratio is less than 2.85 a low risk of developing it in the future if it is not currently present can be predicted.

When there are multiple cut-off values for determining the existence or non-existence of a development risk of Alzheimer's disease, a multistage determination of the existence or non-existence of a development risk of Alzheimer's disease may be made. For example, if the cut-off values are set at A1, A2, A3...An, the ranges of glycoalbumin/hemoglobin A1c (NGSP) ratios can be set at less than A1, A1 to A2, A2 to A3...An-1 to An and above An, and the likelihood that the subject will develop Alzheimer's disease in the future can be predicted to be higher the higher the numerical range. For example, if the cut-off values for determining the existence or non-existence of a development risk of Alzheimer's disease are set at 2.60, 2.85 and 3.00, it can be predicted that "when the glycoalbumin/hemoglobin A1c (NGSP) ratio measured in a biological sample is at least 3.00, there is a extremely high likelihood of developing Alzheimer's disease in the future", while "when it is at least 2.85 and less than 3.00, there is a high likelihood of developing it in the future", "when it is at least 2.60 and less than 2.85, there is a possibility of developing it in the future", and "when it is less than 2.60, there is a low likelihood of developing it in the future".

(5) Device

The device for determining the existence or non-existence of a development risk of Alzheimer's disease may be any device provided with a glycoalbumin measurement part, a hemoglobin A1c measurement part, an operation unit for calculating a measured glycoalbumin/hemoglobin A1c ratio, and an evaluation part for making predictions based on the calculated glycoalbumin/hemoglobin A1c ratio. The measurement parts, operation unit, evaluation part and the like constituting this device do not all have to be installed in the same device, and may each be separate units.

The glycoalbumin measurement part is a site for measuring glycoalbumin in a biological sample introduced into the device. The hemoglobin A1c measurement part is a site for measuring hemoglobin A1c in a biological sample introduced into the device. The sizes and compositions of these sites are not particularly limited, and the measurements are not particularly limited as long as the respective proteins can be measured in a biological sample, and for example the common glycoalbumin and hemoglobin A1c measurement methods described above may be used.

In addition to HPLC methods, immune methods and enzymatic methods, electrophoresis methods, electrode methods, column methods and the like may also be used for measuring glycoalbumin, and other methods may also be used. For example, glycoalbumin may be measured by a widely-used enzymatic method. In enzymatic methods, for example the glycoalbumin in a target sample is first hydrolyzed with a protease, after which the hydrogen peroxide produced by reacting ketoamine oxidase with glycated lysine is reacted with a dye and quantified to determine the glycoalbumin concentration, while the albumin concentration in the same target sample is measured, and the glycoalbumin concentration is divided by the albumin concentration to obtain the glycoalbumin value (% or mmol/mol), but other commercial reagents and known methods and reagents may be used. The measured values for glycoalbumin in the present invention are based on HPLC methods, but values standardized by the ID/MS method or other values may be used, and the cut-off values for these can be converted to the values of the present invention using the relational formulae for the respective values.

A widely used enzymatic method, HPLC method, immune method, column method or electrode method may also be used to measure hemoglobin A1c. In the HPLC method, hemoglobin A1c can be separated and fractionated with an ionexchange column for example, and the concentration (%) calculated. NGSP values are used for the hemoglobin A1c measurements in the present invention, but IFCC or other values may also be used, and the cut-off values and the like can be converted to the values of the present invention using the relational formulae for the respective values.

The operation unit is a site for dividing the glycoalbumin value obtained by measurement in the glycoalbumin measurement part by the hemoglobin A1c value obtained by measurement in the hemoglobin A1c measurement part, and may be a central processing unit (CPU) provided as part of the device for example. After measurement and prior to the operation, the glycoalbumin value and hemoglobin A1c value may also be stored in a storage device such as a memory or hard disk provided as part of the device, or the divided value from the operation unit may be stored temporarily in such a storage device.

The evaluation part is a site for determining the existence or non-existence of a development risk of Alzheimer's disease based on the glycoalbumin/hemoglobin A1c ratio obtained by the calculation part.

In the present device, a determination of the existence or non-existence of a development risk of Alzheimer's disease can be made by comparing the glycoalbumin/hemoglobin A1c ratio obtained by the calculation part with a cut-off value for determining the existence or non-existence of a development risk of Alzheimer's disease. The cut-off value for determining the existence or non-existence of a development risk of Alzheimer's disease may have already been stored in the device, or may be input through an input part of the device when making a prediction. If for example the comparison shows that the glycoalbumin/hemoglobin A1c level obtained by the calculation part is at or above the cut-off value for determining the existence or non-existence of a development risk of Alzheimer's disease, it can be predicted that the individual who provided the biological sample is likely to be at high risk of developing Alzheimer's disease in the future, while conversely if the level is below the cut-off value for determining the existence or non-existence of a development risk of Alzheimer's disease, it can be predicted that the individual who provided the biological sample is at low risk of developing Alzheimer's disease in the future. One cut-off value or multiple values for determining the existence or non-existence of a development risk of Alzheimer's disease may be set in the device, and when multiple cut-off values have been set a multi-stage prediction can be made as described above.

The device may also have an output part. The output part performs processing such as displaying or outputting the results of the determination of the existence or non-existence of a development risk of Alzheimer's disease on a display device such as a screen or on a printing device such as a printer.

From the standpoint of convenience, the device is preferably a POC testing device (in general, a device for testing immediately near the patient in a clinical setting, such as at the bedside). It may also be a handy measurement device which the patient can use at home.

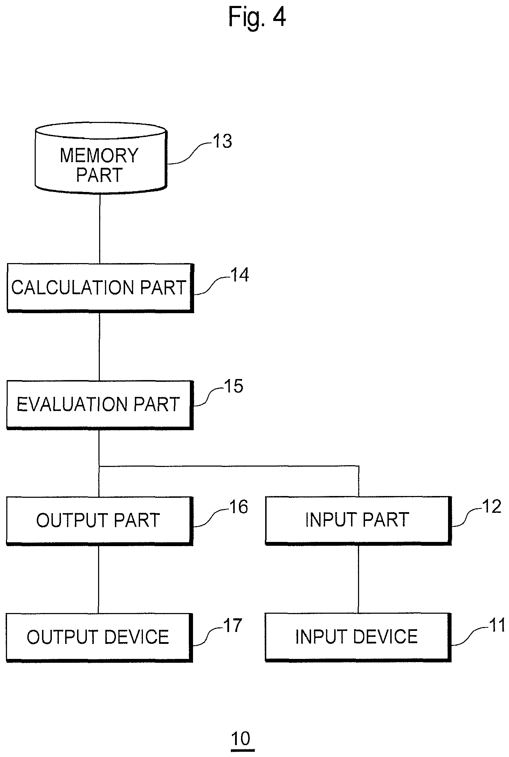

Fig. 4 is a block diagram showing the functional configuration of a device 10 for determining the existence or non-existence of a development risk of Alzheimer's disease in the present invention. As shown in the diagram, the device 10 is provided with an input device 11, an input part 12, a memory part (first memory part, second memory part) 13, a calculation part (first calculation part, second calculation part) 14, an evaluation part 15, an output part 16 and an output device 17.

The device 10 may for example be a specific program executed on a general-purpose personal computer. The input part 12, calculation part 14, evaluation part 15 and output part 16 thus represent operation modules executed by the computer's processor according to the program, and in fact these together constitute the processor of the device 10 of the present invention.

The memory part 13 is a memory device such as a hard disk of the device 10. The input device 11 is an input means such as a keyboard, mouse, touch panel or the like whereby the user sends processing instructions to the device 10 or inputs data and parameters. Data can also be read from a memory medium or the like via a USB (universal serial bus) interface. The operations performed by the user via the input device 11 are controlled by the input part 12. The output device 17 is a display device, printer or the like. Output processing to the output device 17 is controlled by the output part 16.

The user uses the input device 11 to input the glycoalbumin values of blood samples collected from subjects and the hemoglobin A1c values of blood samples collected from subjects.

The memory part 13 records the input glycoalbumin values and hemoglobin A1c values, but may also record the arithmetic expressions and algorithms used in evaluation by the operations part and the calculation results and the like.

The calculation part 14 calculates the glycoalbumin/hemoglobin A1c ratio.

The glycoalbumin/hemoglobin A1c ratio calculated by the calculation part 14 is output to the output device 17 via the output part 16.

The evaluation part 15 evaluates whether or not the glycoalbumin/hemoglobin A1c ratio calculated by the calculation part 14 exceeds a specific threshold. When the evaluation part 15 judges that the glycoalbumin/hemoglobin A1c ratio is at or above a specific threshold, an evaluation result to the effect that the subject may be at risk of developing Alzheimer's disease is output to the output device 17 via the output part 16.

The glycoalbumin measurement reagent contained in the kit for determining the existence or non-existence of a development risk of Alzheimer's disease is not particularly limited as to the measurement method (immune method, enzymatic method, chemical method, etc.) or constitution of the reagent as long as the reagent can measure glycoalbumin in a biological sample. An enzymatic method or immune method is desirable as the measurement method, and for example a Lucica® GA-L (Asahi Kasei Pharma Corporation) reagent using enzymatic methods is a preferred embodiment of the glycoalbumin measurement reagent.

The hemoglobin A1c measurement reagent contained in the kit for determining the existence or non-existence of a development risk of Alzheimer's disease is not particularly limited as to the measurement method (immune method, enzymatic method, etc.) or constitution of the reagent as long as the reagent can measure hemoglobin A1c in a biological sample. Hemoglobin A1c measurement has been standardized by the Japan Diabetes Society and related societies, and measurements confirming to these standards are preferred. For example, CinQ HbA1c (Arkray, Inc.) is an example using an enzymatic method.

The kit for determining the existence or non-existence of a development risk of Alzheimer's disease is used to determine the existence or non-existence of a development risk of Alzheimer's disease using as an indicator the ratio (glycoalbumin/hemoglobin A1c ratio) of the glycoalbumin measured in a biological sample with the glycoalbumin measurement reagent contained in the kit to the hemoglobin A1c measured in a biological sample with the hemoglobin A1c measurement reagent contained in the kit. As discussed above, the risk is determined by comparing the ratio to a cut-off value for determining the existence or non-existence of a development risk of Alzheimer's disease.

**The** biological sample to be measured in the present invention is a blood sample. Preferred examples of test liquids include blood components such as serum, plasma, blood cells, whole blood, or isolated red blood cells or the like. Serum or plasma that has been separated by ordinary methods may also be used. The biological sample may be easily obtained by well-known methods or the like, and may also be a biological sample that has been pre-treated by ordinary methods.

The present disclosure further describes, but not being part of the invention, a preventative and/or treatment agent for dementia or Alzheimer's disease, containing as an active ingredient a substance selected from the group consisting of the diabetes treatment drugs (hypoglycemic drugs, insulin resistance improving drugs, etc.) that has the effect of at least suppressing a rise in the glycoalbumin/hemoglobin A1c ratio.

The present disclosure also describes an agent that inhibits oxidative stress, containing as an active ingredient a substance that has the effect of suppressing production of at least one substance selected from the group consisting of glycoalbumin, hemoglobin A1c and the glycoalbumin/hemoglobin A1c ratio.

The present disclosure also describes an agent that lowers blood glucose, containing as an active ingredient a substance that has the effect of suppressing the expression of at least one selected from the group consisting of glycoalbumin, hemoglobin A1c and the glycoalbumin/hemoglobin A1c ratio.

These agents may be used as drugs or as reagents for experimental purposes.

Examples of the substance that has the effect of suppressing the expression of at least one selected from the group consisting of glycoalbumin, hemoglobin A1c and the glycoalbumin/hemoglobin A1c ratio include uric acid, uric acid lowering drugs such as xanthine oxidase inhibitors, hypoglycemic drugs such as Metformin, SGLT2 inhibitors, Pioglitazone and DPP4 inhibitors, and insulin resistance improvers. Various compounds that promote pancreatic β cell proliferation, and insulin and insulin derivatives, may also be used in the same way.

One kind of substance that has the effect of suppressing the expression of at least one selected from the group consisting of glycoalbumin, hemoglobin A1c and the glycoalbumin/hemoglobin A1c ratio may be used, or multiple kinds may be combined.

A substance having the effect of suppressing the expression of at least one selected from the group consisting of glycoalbumin, hemoglobin A1c and the glycoalbumin/hemoglobin A1c ratio may be administered orally or non-orally to a mammal (such as a human, rabbit, dog, cat, rat or mouse), either alone or as a pharmaceutical composition in an appropriate dosage form. The dosage differs depending on the administration target, type of disease, symptoms, administration route and the like, but for example in the case of an adult, the substance that has the effect of suppressing the expression of at least one selected from the group consisting of glycoalbumin, hemoglobin A1c and the glycoalbumin/hemoglobin A1c ratio may be administered in the amount of normally about 0.1 to 100 mg/kg body weight, or preferably about 0.5 to 50 mg/kg body weight as a single dose about 1 to 2 times per month, and preferably the same dose can be administered for 2 to 3 days consecutively at the beginning of treatment. A corresponding dose can be administered in the case of other non-oral or oral administration. The dose can also be increased according to the symptoms when the symptoms are particularly severe.

A composition for oral administration may be in a solid or liquid dosage form, and specific examples include tablets (including sugar-coated tablets and film-coated tablets), pills, granules, powders, capsules (including soft capsules), syrups, emulsions, suspensions and the like. Such a composition may be manufactured by ordinary methods, and may contain carriers, diluents or excipients commonly used in the pharmaceutical field. Examples of pharmaceutical carriers and excipients include lactose, starch, sucrose, magnesium stearate and the like.

Examples of compositions for non-oral administration include injections, suppositories and the like, and an injection may be in the form of an intravenous injection, subcutaneous injection, intradermal injection, intramuscular injection, drip injection or the like. Such an injection can be prepared by dissolving, suspending or emulsifying a substance that has the effect of suppressing the expression of at least one selected from the group consisting of glycoalbumin, hemoglobin A1c and the glycoalbumin/hemoglobin A1c ratio in a sterile aqueous or oily liquid commonly used for injections. Examples of aqueous liquids for injection include physiological saline and isotonic liquids containing glucose and other adjuvants, and a suitable solubilizing agent such as an alcohol (such as ethanol), polyalcohol (such as propylene glycol or polyethylene glycol), nonionic surfactant (such as Polysorbate 80 or HCO-50 (polyoxyethylene (50 mol) adduct of hydrogenated castor oil)) or the like may also be included. Examples of oily liquids include sesame oil and soy bean oil, and benzyl benzoate, benzyl alcohol or the like may also be included as a solubilizing agent. The prepared injection is filled in a suitable common ampoule. A suppository for rectal administration can be prepared by mixing the substance having the effect of suppressing Smad1 expression with a suitable suppository base.

This oral or non-oral drug composition can be prepared in a unit dosage form suited to the administered dose of the active ingredient. Examples of unit dosage forms include tablets, pills, capsules, injections (ampoules), suppositories and the like.

Moreover, this drug composition may also contain other active ingredients to the extent that these do not cause unwanted interactions when compounded with the substance that has the effect of suppressing the expression of at least one selected from the group consisting of glycoalbumin, hemoglobin A1c and the glycoalbumin/hemoglobin A1c ratio.

To screen candidate compounds from clinical studies, the glycoalbumin/hemoglobin A1c ratio is diagnosed in target cases of diabetes, impaired glucose tolerance (IGT) and impaired fasting glycemia (IFG), the administered drugs and environmental factors such as exercise and diet are tracked throughout the investigation period, and the glycoalbumin/hemoglobin A1c ratio is measured for a period of at least a year after the start of follow-up. Drugs that are shown by frequency method analysis to lower the glycoalbumin/hemoglobin A1c level in these target cases are subjected to drug repositioning and evaluated as compounds exhibiting effectiveness in treating or preventing dementia or Alzheimer's disease, or as seed compounds for potential use as active ingredients in drugs for treating or preventing dementia or Alzheimer's disease.

The present invention is explained in more detail below using examples, but the present invention is not limited to these examples.

Examples

Example 1

Hazard Ratios for Alzheimer's disease Onset According to Levels of Blood Glucose-Associated Indicators

Of the residents aged 65 and older who received a cardiovascular checkup in Hisayama-machi in 2007, 1,187 individuals without dementia were followed prospectivelyf for 5 years. The subjects were separated into 4 quartiles (Q1 to Q4) according to their hemoglobin A1c, glycoalbumin and 1,5-anhydroglucitol levels and glycoalbumin/hemoglobin A1c ratios. The end point was the onset of Alzheimer's disease or vascular dementia. The Cox proportional hazard model was used to calculate the hazard ratio (HR). Alzheimer's disease was diagnosed by the diagnostic criteria of the National Institute of Neurological and Communicative Disorders and Stroke and the Alzheimer's Disease and Related Disorders Association (NINCDS-ADRDA) (Non-Patent Document 2: Guy McKhann et al., Clinical diagnosis of Alzheimer's disease, Neurology 1984, 34:939-944), and vascular dementia by the clinical standard of the National Institute of Neurological Disorders and Stroke and Association Internationale pour la Recherche et l'Enseignement en Neurosciences (NINDS-AIREN) (Roman GC, Tatemichi TK, Erkinjuntti T et al., Vascular dementia: diagnostic criteria for research studies: report of the NINDS-AIREN International Workshop, Neurology 1993, 43:250-260).

The following conditions 1) to 4) must be met in order for a subject to be diagnosed with Alzheimer's disease according to the NINCDS-ADRDA diagnostic criteria.

1) Meets the diagnostic criteria for clinical examination (probable Alzheimer's disease)

2) Progressive impairment or the like of specific cognitive function is confirmed, and findings support Alzheimer's disease

3) There are other clinical features consistent with probable Alzheimer's disease once causes of dementia other than Alzheimer's disease have been excluded

4) Cases in which probable Alzheimer's disease diagnosis is doubtful, or patients with features that do not match probable Alzheimer's disease are excluded from probable Alzheimer's disease diagnosis

In this study, cranial imaging (CT/MRI) was also performed to obtain findings (see 2 above) supporting the NINCDS-ADRDA diagnosis of probable Alzheimer's disease. Specifically, patients who exhibited no clear organic lesions typical of cerebral vascular disease and who exhibited cerebral atrophy (especially internally, in the temporal and parietal lobe regions) were diagnosed with probable Alzheimer's disease.

91.4% of the Alzheimer's disease diagnoses in the current study were cases of probable Alzheimer's disease according to NINCDS-ADRDA, and included some subjects who died during the follow-up period and received a conclusive diagnosis of definite Alzheimer's disease based on an autopsy. That is, the "91.4%" are subjects who:

1) Fulfill the diagnostic criteria for clinical examination (probable Alzheimer's disease), that is:

- Dementia found in clinical testing and testing by the Mini-Mental State Examination (MMSE), Blessed Dementia Scale or similar, and confirmed by neuropsychological testing

- Deficits in two or more cognitive areas

- Progressive decline in memory and other cognitive functions

- Consciousness not impaired

- Onset at age 40 to 90, most common after age 65

- Progressive memory and cognitive impairment not attributable to any other system or brain disease.

2) Supporting findings are:

- Progressive impairment in specific cognitive functions: language impairment (aphasia), behavioral impairment (apraxia), impaired recognition (agnosia) and impairment of other activities of daily living (ADL), changes in behavioral patterns, family history of similar impairment, especially when there is neuropathological confirmation, and

- As a clinical test finding, brain atrophy or the like is confirmed by "cranial imaging", or in other words by CT/MIR or the like.

3) There are other clinical features consistent with the diagnosis of probable Alzheimer's disease once causes of dementia other than Alzheimer's disease have been excluded

4) There are no features that call the diagnosis of probable Alzheimer's disease into question, or that are not characteristic of probable Alzheimer's disease.

Regarding the age range given in the 1) diagnostic criteria for clinical examination (probable Alzheimer's disease), this was re-interpreted as "onset at age 40 or above" for purposes of the diagnosis because subjects who may have developed Alzheimer's disease at age 90 or above could be included.

Finally, probable Alzheimer's disease was diagnosed after a detail examination of the subject's medical history and symptomology.

The remaining 8.6% of subjects diagnosed with Alzheimer's disease were a group of subjects who could not be examined by cranial imaging, but who were diagnosed with Alzheimer's disease based on medical history and symptomology (current symptoms), and who came very close to meeting the NINCDS-ADRDA criteria for probable Alzheimer's disease. More specifically, although clinical test findings have been established as supporting findings for a NINCDS-ADRDA diagnosis of probable Alzheimer's disease, the "8.6%" were those who were diagnosed with Alzheimer's disease after a detailed investigation of "medical history" and "symptomology" because MRI imaging could not be performed even though they fulfilled all the other conditions. "Medical history" here means the history of previous diseases and the history of the current disease. Symptoms here mean pathological changes appearing in the body, and symptomatology in the case of Alzheimer's disease symptomology is a field of medicine and is a methodology for defining, classifying and interpreting the various complaints and medical examination findings (and symptoms) of a patient.

In other words, the most important factors in Alzheimer's disease diagnosis are "medical history" and "symptomology" (symptoms displayed), and the collecting of these is emphasized in the present study. Moreover, other clinical data was collected as much as possible and cranial imaging was performed to support the diagnosis of probable Alzheimer's disease, and when the subject died an autopsy was performed to obtain a final diagnosis of definite Alzheimer's disease, thereby completing a 3-stage Alzheimer's disease diagnosis.

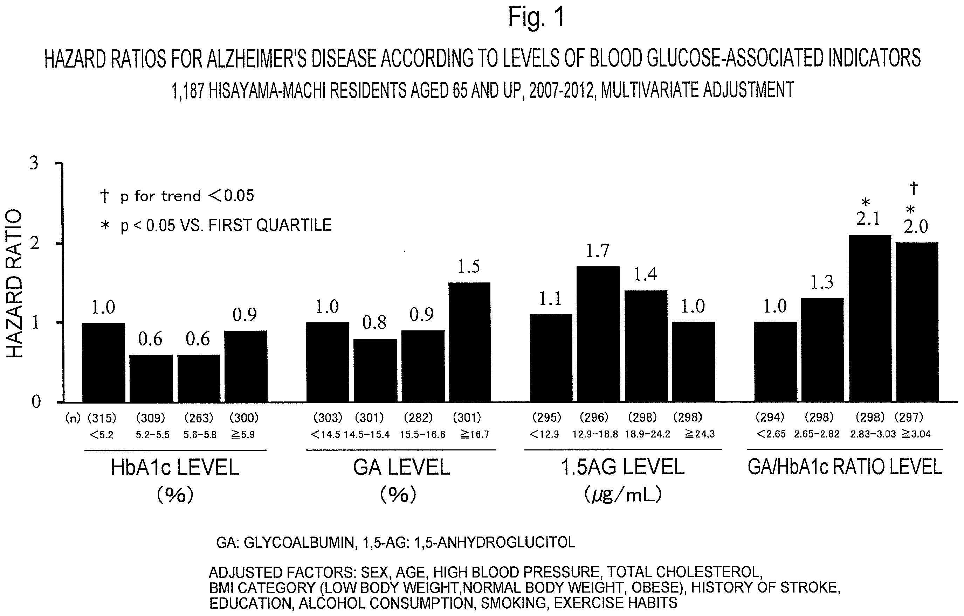

During the follow-up period 116 subjects developed Alzheimer's disease, and 43 developed vascular dementia. The incidences (sex- and age-adjusted) of Alzheimer's disease and vascular dementia were investigated against the levels of each blood glucose-associated indicator. The results show that the Alzheimer's disease incidence rose significantly as the glycoalbumin/hemoglobin A1c levels rose, and although not significant, the same tendency was shown for glycoalbumin. However, no clear relationship was shown for hemoglobin A1c and 1,5-anhydroglucitol. No relationship was shown between any of these blood glucose-associated indicators and the incidence of vascular dementia. In a multivariate analysis, adjusting the variables of sex, age, high blood pressure, total cholesterol, body mass index (BMI, obesity) category (low body weight, normal body weight, obese), history of stroke, education, alcohol consumption, smoking and exercise habits had no effect on the significant positive relationship between glycoalbumin/hemoglobin A1c level and Alzheimer's disease (trend $p = 0.01$), and the hazard ratio for Alzheimer's disease incidence was significantly higher in both the Q3 group (2.1) and Q4 group (2.0) in comparison with the Q1 group (Fig. 1, $p < 0.05$).

These results show that the glycoalbumin/hemoglobin A1c ratio is a biomarker associated with the incidence of or the existence or non-existence of a development risk of Alzheimer's disease, and that the hazard of developing Alzheimer's disease is significantly higher when the glycoalbumin/hemoglobin A1c level is 2.83 or more, or in other words that the incidence of or the existence or non-existence of a development risk of Alzheimer's disease can be determined using the glycoalbumin/hemoglobin A1c level as an indicator.

This also shows that the risk of developing Alzheimer's disease can be determined based on measurement results obtained with a commercial glycoalbumin measurement kit and hemoglobin A1c measurement kit or device, and that a kit or device for determining the risk of Alzheimer's disease can be provided.

Example 2

Hazard ratios of Blood Glucose-associated Indicator Levels for Alzheimer's Disease According to Presence or Absence of Abnormal Glucose Metabolism

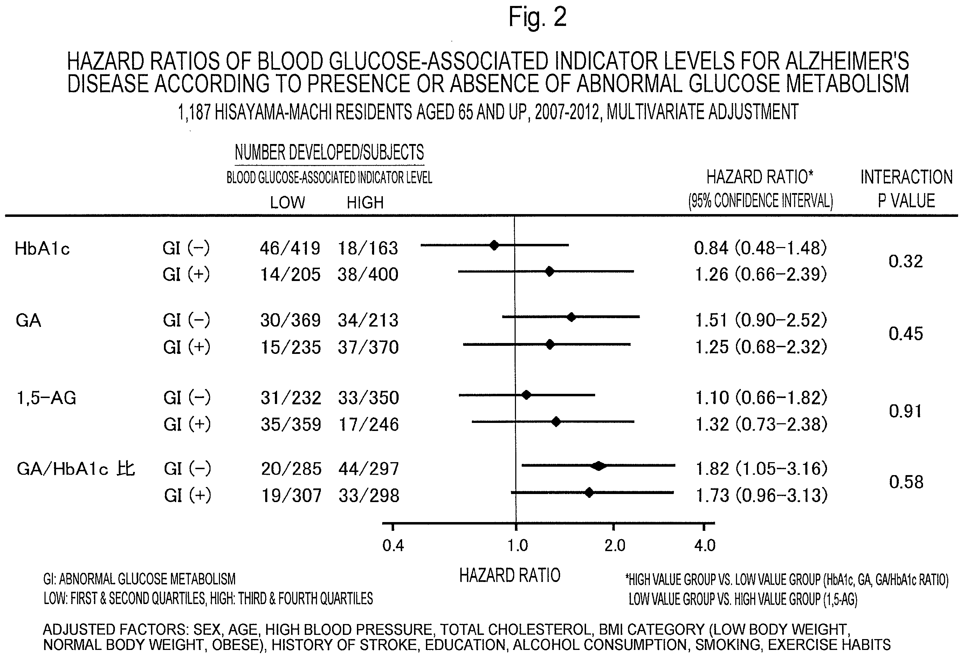

The hazard ratios (after multivariate adjustment) of each blood glucose-associated indicator level in Alzheimer's disease onset were calculated depending on the presence or absence of abnormal glucose metabolism (diabetes + prediabetes) under the same conditions as in Example 1. As a result, in the group without abnormal glucose metabolism the high glycoalbumin/hemoglobin A1c ratio groups (Q3 and Q4) had significantly higher hazard ratios (1.82, $p = 0.03$) for Alzheimer's disease onset than the low-ratio groups (Q1 and Q2), and the same tendency was seen in the group with abnormal glucose metabolism (Fig. 2, hazard ratio 1.7, $p = 0.07$). However, no significant relationship was found between Alzheimer's disease onset and hemoglobin A1c, glycoalbumin or 1,5-anhydroglucitol regardless of whether there was abnormal glucose metabolism ($p > 0.1$ in all cases). There were no interactions between the blood glucose-associated indicator levels and abnormal glucose metabolism.

These results show that the glycoalbumin/hemoglobin A1c ratio can be used to determine the incidence of or the existence or non-existence of a development risk of Alzheimer's disease even in subjects with no abnormal glucose metabolism.

Because the incidence of or the existence or non-existence of a development risk of Alzheimer's disease can be determined based on measurement results obtained with a commercial glycoalbumin measurement kit or device and a commercial hemoglobin A1c measurement kit or device as in Example 1, the present invention can clearly provide the use of a kit and device for determining the existence or non-existence of a development risk of Alzheimer's disease.

Example 3

Calculating Cut-off Values for Glycoalbumin/Hemoglobin A1c Levels in Alzheimer's Disease Onset

Cut-off values and sensitivity/specificity of glycoalbumin and glycoalbumin/hemoglobin A1c levels were determined for Alzheimer's disease onset using ROC (receiver operating characteristics curve) analysis under the same conditions as in Example 1. The results are shown in Table 1.

[Table 1]

Table 1: Cut-off values for blood glucose-associated indicators in Alzheimer's disease onset
(1,187 Hisayama residents aged 65 and older, 2007-2012, ROC analysis)

| BG-associated indicator | Cut-off value | Sensitivity (%) | Specificity (%) |
| --- | --- | --- | --- |
| GA | 15.8% | 55.2% | 59.9% |
| GA/HbA1c | 2.85 | 65.5% | 53.3% |

**As** shown in Table 1, the glycoalbumin/hemoglobin A1c ratio cut-off value for detecting Alzheimer's disease is 2.85, with a sensitivity of 65.5% and a specificity of 53.3%. These results show that the glycoalbumin/hemoglobin A1c ratio is a biomarker for Alzheimer's disease, and that the presence or absence of Alzheimer's disease can be predicted or the existence or non-existence of a development risk of Alzheimer's disease can be determined using a cut-off value for detecting the incidence of or the existence or non-existence of a development risk of Alzheimer's disease, or in other words that many tests can be performed at once easily and cheaply without the advanced and complex diagnosis and testing used in the past.

This also shows, similarly to Example 1, that the risk of developing Alzheimer's disease can be determined based on measurement results obtained with a commercial glycoalbumin measurement kit or device and a commercial hemoglobin A1c measurement kit or device, and that use of a kit or device for determining the existence or non-existence of a development risk of Alzheimer's disease can be provided by the present invention.

In this Example 3, the presence or absence of Alzheimer's disease can be predicted using the glycoalbumin/hemoglobin A1c ratio as an indicator for example. Specifically, if the glycoalbumin/hemoglobin A1c ratio is at least 2.85, the likelihood that Alzheimer's disease is present can be judged to be high, while if it is less than 2.85 the likelihood can be judged to be low. If it is at least 2.85, the patient can then be diagnosed with Alzheimer's disease using brain imaging requiring expensive equipment, invasive cerebrospinal fluid testing, or the complex diagnostic criteria of NINCDS-ADRDA or the like, and treatment can be initiated as necessary. This means that with the present invention the likelihood of Alzheimer's disease can be predicted easily and cheaply without the need for brain imaging requiring expensive equipment, invasive cerebrospinal fluid testing, or elaborate and complex Alzheimer's disease diagnoses.

Moreover, even if a subject has been judged not to have Alzheimer's disease based on such a detailed Alzheimer's disease diagnosis, a subject with a glycoalbumin/hemoglobin A1c ratio of at least 2.85 has a high likelihood of developing Alzheimer's disease in the future. Consequently, with the present invention the likelihood of developing Alzheimer's disease in the future can be predicted and future onset can be prevented easily and cheaply without using brain imaging requiring expensive equipment, invasive cerebrospinal fluid testing, or the complex diagnostic criteria of NINCDS-ADRDA or the like, and patients can receive necessary health guidance and take measures to prevent onset.

Industrial Applicability

In the present invention, the existence or non-existence of a development risk of Alzheimer's disease can be determined easily, rapidly and cheaply by measuring glycoalbumin and hemoglobin A1c and determining the glycoalbumin/hemoglobin A1c ratio.

The method of the present invention is useful for clinical testing is useful for risk assessment of Alzheimer's disease.

Reference Signs List

| | |
|---|---|
| 10 | Blood sample device |
| 11 | Input device |
| 12 | Input part |
| 13 | Memory part |
| 14 | Calculation part |
| 15 | Evaluation part |
| 16 | Output part |
| 17 | Output device |

**Claims**

1. A method for determining the existence or non-existence of a development risk of Alzheimer's disease, comprising a step of determining that a subject is at risk of developing Alzheimer's disease when a glycoalbumin/hemoglobin A1c ratio in a blood sample from the subject is at least 2.6.

2. The method according to Claim 1, wherein the blood sample is subjected to the following steps (1) to (3) prior to the step of determining the existence or non-existence of a development risk of Alzheimer's disease:

   1) measuring glycoalbumin and hemoglobin A1c in the blood sample from the subject;
   2) using the measurement values obtained in step 1) to calculate the glycoalbumin/hemoglobin A1c ratio;
   3) comparing the glycoalbumin/hemoglobin A1c ratio obtained in step 2) with a cut-off value for predicting the existence or non-existence of a development risk of Alzheimer's disease.

3. A method according to Claim 1 or 2, wherein the glycoalbumin/hemoglobin A1c ratio is at least 2.85.

4. A method according to any one of Claims 1 to 3, wherein the subject does not suffer from mild cognitive impairment (MCI).

5. A method according to any one of Claims 1 to 4, wherein the subject does not suffer from vascular dementia.

6. Use of a biomarker for determining the existence or non-existence of a development risk of Alzheimer's disease, wherein the biomarker is a glycoalbumin/hemoglobin A1c ratio, and wherein a subject is determined to be at risk of developing Alzheimer's disease when the glycoalbumin/hemoglobin A1c ratio in a blood sample from the subject is at least 2.6.

7. Use of a device in a method for determining the existence or non-existence of a development risk of Alzheimer's disease according to any one of claims 1 to 5, wherein the device has:

   (a) a calculation unit for calculating a glycoalbumin/hemoglobin A1c ratio based on measurement values for glycoalbumin and hemoglobin A1c; and
   (b) an evaluation part for evaluating the existence or non-existence of a development risk of Alzheimer's disease based on the calculated glycoalbumin/hemoglobin A1c ratio.

8. The use of a device according to Claim 7, wherein the device further has a measuring part for glycoalbumin and/or a measuring part for hemoglobin A1c.

9. Use of a kit in a method for determining the existence or non-existence of a development risk of Alzheimer's disease according to any one of claims 1 to 5, wherein the kit comprises a glycoalbumin measurement reagent and a hemoglobin A1c measurement reagent, and a manual stating to the effect that a subject is determined to be at risk of developing Alzheimer's disease when a glycoalbumin/hemoglobin A1c ratio is at least 2.6.

**Patentansprüche**

1. Verfahren zur Bestimmung des Vorhandenseins oder Nichtvorhandenseins des Risikos der Entwicklung der Alzheimer-Krankheit, umfassend einen Schritt der Bestimmung, dass ein Subjekt das Risiko aufweist, die Alzheimer-Krankheit zu bekommen, wenn das Glykoalbumin/Hämoglobin Ale-Verhältnis in einer Blutprobe des Subjekts mindestens 2,6 beträgt.

2. Verfahren nach Anspruch 1, wobei die Blutprobe vor dem Schritt der Bestimmung des Vorhandenseins oder Nichtvorhandenseins des Risikos der Entwicklung der Alzheimer-Krankheit den folgenden Schritten (1) bis (3) unterzogen wird:

   1) Messung von Glykoalbumin und Hämoglobin Ale in der Blutprobe des Subjekts;
   2) Verwendung der in Schritt 1) erhaltenen Messwerte zur Berechnung des Verhältnisses Glykoalbumin/Hämoglobin A1c;
   3) Vergleich des in Schritt 2) erhaltenen Glykoalbumin/Hämoglobin A1c-Verhältnisses mit einem Grenzwert zur Vorhersage des Vorhandenseins oder Nichtvorhandenseins des Risikos der Entwicklung der Alzheimer-Krankheit.

3. Verfahren nach Anspruch 1 oder 2, wobei das Glykoalbumin/Hämoglobin Ale-Verhältnis mindestens 2,85 beträgt.

4. Verfahren nach einem der Ansprüche 1 bis 3, wobei das Subjekt nicht an einer leichten kognitiven Beeinträchtigung (MCI) leidet.

5. Verfahren nach einem der Ansprüche 1 bis 4, wobei der Patient nicht an einer vaskulären Demenz leidet.

6. Verwendung eines Biomarkers zur Bestimmung des Vorhandenseins oder Nichtvorhandenseins des Risikos der Entwicklung der Alzheimer-Krankheit, wobei der Biomarker das Glykoalbumin/Hämoglobin Alc-Verhältnis ist und wobei bestimmt wird, dass ein Subjekt das Risiko der Entwicklung der Alzheimer-Krankheit aufweist, wenn das Glykoalbumin/Hämoglobin Ale-Verhältnis in einer Blutprobe des Subjekts mindestens 2,6 beträgt.

7. Verwendung einer Vorrichtung in einem Verfahren zur Bestimmung des Vorhandenseins oder Nichtvorhandenseins des Risikos der Entwicklung der Alzheimer-Krankheit nach einem der Ansprüche 1 bis 5, wobei die Vorrichtung aufweist:

   (a) eine Berechnungseinheit zur Berechnung des Glykoalbumin/Hämoglobin Alc-Verhältnisses auf Basis von Messwerten für Glykoalbumin und Hämoglobin Alc; und
   (b) einen Bewertungsteil zum Bewerten des Vorliegens oder Nichtvorliegens des Risikos der Entwicklung der Alzheimer-Krankheit auf Basis des berechneten Glykoalbumin/Hämoglobin Alc-Verhältnisses.

8. Verwendung einer Vorrichtung nach Anspruch 7, wobei die Vorrichtung ferner einen Messteil für Glykoalbumin und/oder einen Messteil für Hämoglobin Alc aufweist.

9. Verwendung eines Kits in einem Verfahren zur Bestimmung des Vorhandenseins oder Nichtvorhandenseins des Risikos der Entwicklung der Alzheimer-Krankheit nach einem der Ansprüche 1 bis 5, wobei das Kit ein Glykoalbumin-Messreagenz und ein Hämoglobin-Alc-Messreagenz sowie eine Anleitung umfasst, die besagt, dass bei einem Subjekt das Risiko der Entwicklung der Alzheimer-Krankheit festgestellt wird, wenn das Verhältnis Glykoalbumin/Hämoglobin Alc mindestens 2,6 beträgt.

**Revendications**

1. Procédé pour déterminer l'existence ou la non-existence d'un risque de développement de la maladie d'Alzheimer, comprenant une étape de déterminer qu'un sujet est à risque de développer la maladie d'Alzheimer lorsqu'un rapport glycoalbumine/hémoglobine A1c dans un échantillon de sang du sujet est d'au moins 2,6.

2. Procédé selon la revendication 1, dans lequel l'échantillon de sang est soumis aux étapes (1) à (3) suivantes avant l'étape de détermination de l'existence ou de la non-existence d'un risque de développement de la maladie d'Alzheimer :

1) mesure de la glycoalbumine et de l'hémoglobine A1c dans l'échantillon de sang du sujet ;
2) utiliser les valeurs de mesure obtenues à l'étape 1) pour calculer le rapport glycoalbumine/hémoglobine A1c ;
3) comparer le rapport glycoalbumine/hémoglobine A1c obtenu à l'étape 2) avec une valeur seuil pour prédire l'existence ou la non-existence d'un risque de développement de la maladie d'Alzheimer.

3. Procédé selon la revendication 1 ou 2, dans lequel le rapport glycoalbumine/hémoglobine A1c est d'au moins 2,85.

4. Méthode selon l'une quelconque des revendications 1 à 3, dans laquelle le sujet ne souffre pas de déficience cognitive légère (MCI).

5. Méthode selon l'une quelconque des revendications 1 à 4, dans laquelle le sujet ne souffre pas de démence vasculaire.

6. Utilisation d'un biomarqueur pour déterminer l'existence ou la non-existence d'un risque de développement de la maladie d'Alzheimer, dans laquelle le biomarqueur est un rapport glycoalbumine/hémoglobine A1c, et dans laquelle un sujet est déterminé comme étant à risque de développer la maladie d'Alzheimer lorsque le rapport glycoalbumine/hémoglobine A1c dans un échantillon de sang du sujet est d'au moins 2,6.

7. Utilisation d'un dispositif dans une méthode de détermination de l'existence ou de la non-existence d'un risque de développement de la maladie d'Alzheimer selon l'une quelconque des revendications 1 à 5, dans laquelle le dispositif comporte :

(a) une partie de calcul pour calculer un rapport glycoalbumine/hémoglobine A1c à partir de valeurs de mesure de la glycoalbumine et de l'hémoglobine A1c ; et
(b) une partie d'évaluation pour évaluer l'existence ou la non-existence d'un risque de développement de la maladie d'Alzheimer sur la base du rapport glycoalbumine/hémoglobine A1c calculé.

8. Utilisation d'un dispositif selon la revendication 7, dans laquelle le dispositif comporte en outre une partie de mesure de la glycoalbumine et/ou une partie de mesure de l'hémoglobine A1c.

9. Utilisation d'un kit dans une méthode de détermination de l'existence ou de l'inexistence d'un risque de développement de la maladie d'Alzheimer selon l'une quelconque des revendications 1 à 5, dans laquelle le kit comprend un réactif de mesure de la glycoalbumine et un réactif de mesure de l'hémoglobine A1c, ainsi qu'un manuel indignant qu'un sujet est déterminé comme étant à risque de développer la maladie d'Alzheimer lorsqu'un rapport glycoalbumine/hémoglobine A1c est d'au moins 2,6.

## Fig. 1

### HAZARD RATIOS FOR ALZHEIMER'S DISEASE ACCORDING TO LEVELS OF BLOOD GLUCOSE-ASSOCIATED INDICATORS

1,187 HISAYAMA-MACHI RESIDENTS AGED 65 AND UP, 2007-2012, MULTIVARIATE ADJUSTMENT

GA: GLYCOALBUMIN, 1,5-AG: 1,5-ANHYDROGLUCITOL

ADJUSTED FACTORS: SEX, AGE, HIGH BLOOD PRESSURE, TOTAL CHOLESTEROL,
BMI CATEGORY (LOW BODY WEIGHT, NORMAL BODY WEIGHT, OBESE), HISTORY OF STROKE,
EDUCATION, ALCOHOL CONSUMPTION, SMOKING, EXERCISE HABITS

EP 3 537 155 B1

## Fig. 2

### HAZARD RATIOS OF BLOOD GLUCOSE-ASSOCIATED INDICATOR LEVELS FOR ALZHEIMER'S DISEASE ACCORDING TO PRESENCE OR ABSENCE OF ABNORMAL GLUCOSE METABOLISM

#### 1,187 HISAYAMA-MACHI RESIDENTS AGED 65 AND UP, 2007-2012, MULTIVARIATE ADJUSTMENT

| | | NUMBER DEVELOPED/SUBJECTS | | | HAZARD RATIO* (95% CONFIDENCE INTERVAL) | INTERACTION P VALUE |
|---|---|---|---|---|---|---|
| | | BLOOD GLUCOSE-ASSOCIATED INDICATOR LEVEL | | | | |
| | | LOW | HIGH | | | |
| HbA1c | GI (−) | 46/419 | 18/163 | | 0.84 (0.48−1.48) | 0.32 |
| | GI (+) | 14/205 | 38/400 | | 1.26 (0.66−2.39) | |
| GA | GI (−) | 30/369 | 34/213 | | 1.51 (0.90−2.52) | 0.45 |
| | GI (+) | 15/235 | 37/370 | | 1.25 (0.68−2.32) | |
| 1,5-AG | GI (−) | 31/232 | 33/350 | | 1.10 (0.66−1.82) | 0.91 |
| | GI (+) | 35/359 | 17/246 | | 1.32 (0.73−2.38) | |
| GA/HbA1c 比 | GI (−) | 20/285 | 44/297 | | 1.82 (1.05−3.16) | 0.58 |
| | GI (+) | 19/307 | 33/298 | | 1.73 (0.96−3.13) | |

```
0.4        1.0      2.0       4.0
            HAZARD RATIO
```

GI: ABNORMAL GLUCOSE METABOLISM
LOW: FIRST & SECOND QUARTILES, HIGH: THIRD & FOURTH QUARTILES

*HIGH VALUE GROUP VS. LOW VALUE GROUP (HbA1c, GA, GA/HbA1c RATIO)
LOW VALUE GROUP VS. HIGH VALUE GROUP (1,5-AG)

ADJUSTED FACTORS: SEX, AGE, HIGH BLOOD PRESSURE, TOTAL CHOLESTEROL, BMI CATEGORY (LOW BODY WEIGHT, NORMAL BODY WEIGHT, OBESE), HISTORY OF STROKE, EDUCATION, ALCOHOL CONSUMPTION, SMOKING, EXERCISE HABITS

Fig. 3

RANGE CONFIRMABLE BY MMSE ≈ MOCA ≈ HDS-R (REVISED HASEGAWA'S DEMENTIA SCALE)

MILD COGNITIVE
IMPAIRMENT
(MCI)

DEMENTIA
(DSM–ⅢR)

ALZHEIMER'S DISEASE
(NINCDS–ADRDA)

VASCULAR DEMENTIA
(NINDS–AIREN)

Fig. 4

```
          ┌─────────────┐
          │   MEMORY    │──── 13
          │    PART     │
          └──────┬──────┘
                 │
          ┌──────┴──────────┐
          │ CALCULATION PART │──── 14
          └──────┬──────────┘
                 │
          ┌──────┴──────────┐
          │ EVALUATION PART │──── 15
          └──────┬──────────┘
                 │
         ┌───────┴──────────────────────┐
         │                              │
  ┌──────┴──────┐              ┌────────┴─────┐
  │ OUTPUT PART │──── 16       │  INPUT PART  │──── 12
  └──────┬──────┘              └────────┬─────┘
         │                              │
  ┌──────┴──────┐              ┌────────┴─────┐
  │OUTPUT DEVICE│──── 17       │ INPUT DEVICE │──── 11
  └─────────────┘              └──────────────┘
```

10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011034088 A

- JP 2015096835 A

### Non-patent literature cited in the description

- Guideline for Treatment of Dementia Disease. 010 Guideline for Treatment of Dementia Disease. Japanese Society of Neurology, 15 October 2010
- **GUY MCKHANN et al.** Clinical diagnosis of Alzheimer's disease. *Neurology,* 1984, vol. 34, 939-944
- **TOMOYUKI KOHARA ; YUTAKA KIYOHARA.** Dementia in diabetes patients. *Gekkan Tonyobyo (Diabetes Monthly),* 2012, vol. 4, 12-20
- **IZUMI TAKEI et al.** Recommended JSCC methods for measuring glycoalbumin, Rinshokagaku. *Clinical Chemistry,* 2008, vol. 37, 178-191
- **KWANG JOON KIM et al.** The roles of glycated albumin as intermediate glycation index and pathogenic protein. *Diabetes Metab. J.,* 2012, vol. 36, 98-107

- **YUAN ZHONG et al.** GA to HbA1C ratio, but not HbA1C is associated with cognition in Chinese nondiabetic old adults. *Aging Mental Health,* 2015, vol. 19 (9), 853-857
- **TOMOE KINOSHITA et al.** Association of GA/HbA1c ratio and cognitive impairment in subjects with type 2 diabetes mellitus. *J. Diabetes Complications,* 2016
- **ROMAN GC ; TATEMICHI TK ; ERKINJUNTTI T et al.** Vascular dementia: diagnostic criteria for research studies: report of the NINDS-AIREN International Workshop. *Neurology,* 1993, vol. 43, 250-260